# EUROPÄISCHE PATENTANMELDUNG

(11) **EP 1 138 675 A2**
(43) Veröffentlichungstag der Anmeldung: **04.10.2001**
(21) Anmeldenummer: 01106525.7
(22) Anmeldetag: 15.03.2001
(51) Int. Cl.: C07D 239/42

(54) **Verfahren zur Herstellung von 2-Methyl-4-amino-5-aminomethylpyrimidin**

(30) Priorität: 29.03.2000 DE 10015470
(71) Anmelder: BASF AKTIENGESELLSCHAFT, 67056 Ludwigshafen (DE)
(72) Erfinder: Ernst, Hansgeorg, Dr., 67346 Speyer (DE); Frauenkron, Matthias, Dr., 67061 Ludwigshafen (DE); Melder, Johann-Peter, Dr., 67459 Böhl-Iggelheim (DE); Funke, Frank, Dr., 67227 Frankenthal (DE); Keller, Andreas, 76726 Germersheim (DE)

(57) **Zusammenfassung**

Verfahren zur Herstellung von 2-Methyl-4-amino-5-aminomethylpyrimidin der Formel 1 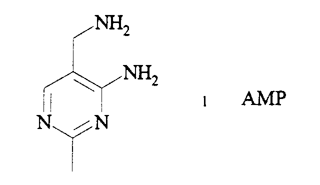 durch Umsetzung von 2-Methyl-4-amino-5-alkoxymethylpyrimidin der Formel 2 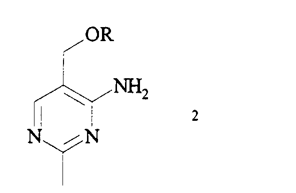 mit R = C₁-C₆-Alkyl
mit Ammoniak in Gegenwart eines Katalysators.

## Beschreibung

Die vorliegende Erfindung betrifft ein verbessertes Verfahren zur Herstellung von 2-Methyl-4-amino-5-aminomethylpyrimidin der Formel 1 (im folgenden kurz AMP genannt), 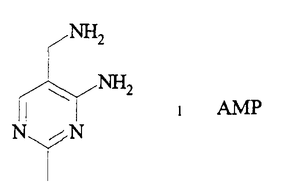 ausgehend von einem 2-Methyl-4-amino-5-alkoxymethylpyrimidin der Formel 2, mit

R gleich C₁-C₆ Alkyl.

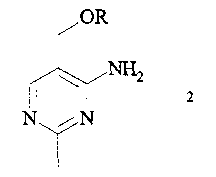

Das Derivat der Formel 2 mit R = Methyl wird im folgenden als MMP ('Methoxymethylpyrimidin') bezeichnet.

AMP 1 ist das zentrale Zwischenprodukt aller technisch relevanten Verfahren für die Herstellung von Vitamin B 1 (Thiamin). Da der Bedarf an diesem Vitamin ständig ansteigt, sind schon viele Versuche unternommen worden, ein vorteilhaftes Verfahren zur Herstellung des Pyrimidin-Teils von Thiamin zu entwickeln.

Eine aktuelle und umfassende Darstellung dieser Verfahren ist z.B. in Ullmann's Encyclopedia of Industrial Chemistry, Vol. A 27 (1996), pp. 515-517 erschienen. Als Edukte werden generell Acrylnitril, Malodinitril oder Acetonitril eingesetzt.

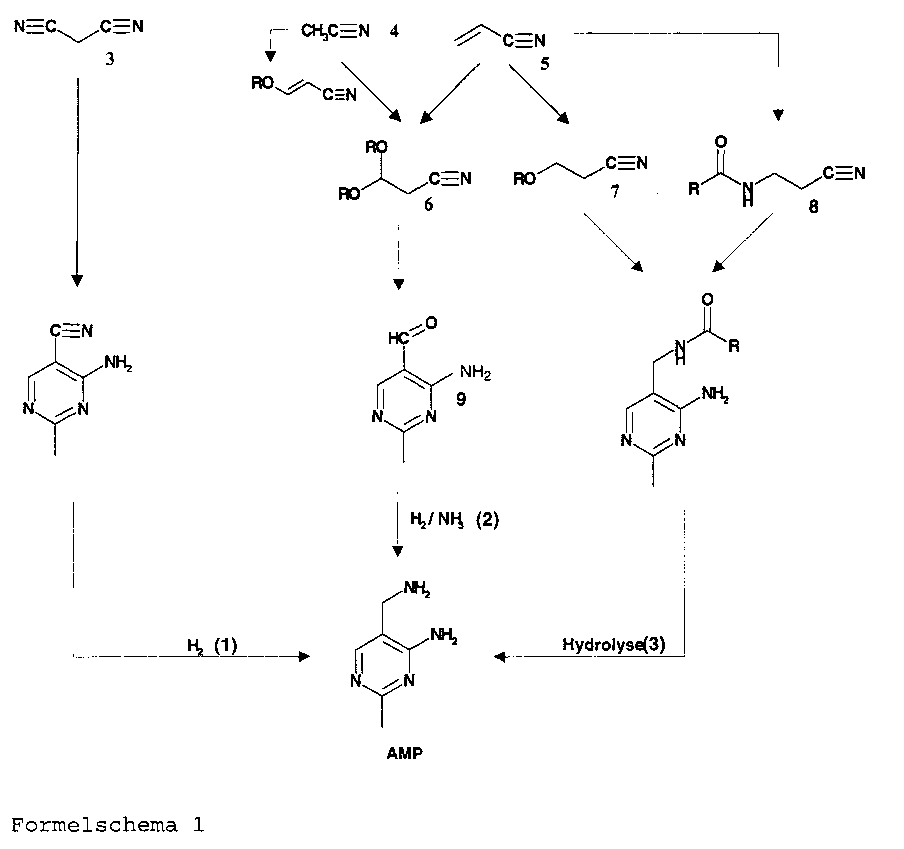

Allen diesen Verfahren ist gemeinsam, daß man nicht direkt zum gewünschten Produkt AMP, sondern grundsätzlich zunächst zu AMP-Derivaten gelangt, die sich entweder im falschen Oxidationszustand befinden oder eine N-Acylfunktion tragen. Bei allen diesen Verfahren muß die Aminomethylseitenkette von AMP durch aufwendige und teure Reduktionsprozesse (Hydrierung einer Nitrilgruppe (1) bzw. reduktive Aminierung einer Formylgruppe (2)) oder durch Hydrolyse der N-Acylgruppe (3) erzeugt werden.

Umwandlungen von funktionellen Gruppen an wertvollen Zwischenstufen gestalten ein Verfahren generell umständlich und damit unökonomisch.

Auch seitens der Edukte sind die bekannten Verfahren ungünstig. Malodinitril 3 ist sehr teuer und sicherheitstechnisch nicht unproblematisch. Der Aufbau des Pyrimidin-Gerüsts ausgehend von Acetonitril 4 ist wegen der zusätzlichen Verfahrensstufen zur Einführung des C₁-Bausteins langwierig und umständlich. Die Synthesewege aus Acrylnitril 5 führen entweder über β-Dialkoxypropionitrile 6, N-funktionalisierte β-Aminopropionitrile 8, oder über β-Alkoxypropionitrile 7.

Der Weg über β-Dialkoxypropionitril 6 beinhaltet die obengenannte umständliche reduktive Aminierung einer Formylpyrimidin-Zwischenstufe.

β-Aminopropionitril andererseits ist eine sicherheitstechnisch nicht unproblematische und zudem sehr toxische Substanz. β-Alkoxypropionitrile sind dagegen praktisch untoxisch (Ullmann's Encyclopädie der technischen Chemie, 4. Auflage, Bd. 17 (1979), S. 330). Die Herstellung von AMP aus β-Alkoxypropionitrilen, die wegen ihrer geringen Toxizität gut zu handhabende Vorprodukte sind, wurde in der Literatur wiederholt beschrieben (s. u.a. Chem. Ber. 106 (1973), 893; Bull. Chem. Soc. Japan 45 (1972), 1127; DE-A 1016266).

Besonders nachteilig an dem bisher durchgeführten Prozeß ist, daß für die Einführung des Aminomethylen-Stickstoffs ein zusätzliches Äquivalent des teuren Acetamidin verbraucht wird. Das resultierende N-Acetyl-AMP muß unter drastischen Bedingungen zum freien Amin verseift werden:

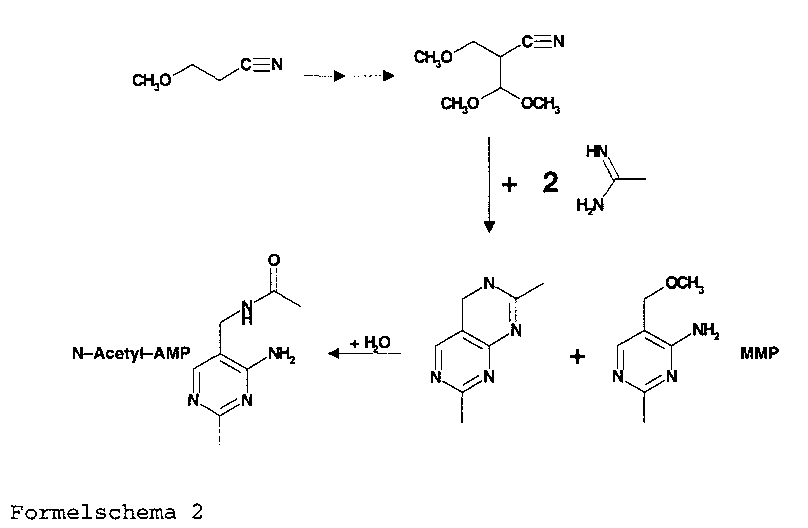

Als Nebenprodukt entsteht bei diesem Verfahren außerdem MMP, das nicht in AMP überführt wird, wodurch die Effizienz des Gesamtprozesses beeinträchtigt wird.

Benzylether des Typs 2 wurden in der Literatur verschiedentlich Etherspaltungen unterworfen (Chim. Ther. 8 (1973) 1, 98; BE 590665; Khim.-Farm. Zh. 23 (1989), 11, 1374; US 3,161,642; GB 953,875). Dabei sind in der Regel drastische Bedingungen erforderlich (starke Mineralsäuren).

Das dabei entstehende korrespondierende Benzylhalogenid wird in einem weiteren Schritt mit NH₃ umgesetzt (Otkrytiya, Izobret., Prom. Obraztsy, Tovarnye Znaki 1969, 46 (8), 22). Die Selektivität der Monoalkylierung von NH₃ mit reaktiven Halogeniden ist bekanntlich schlecht. Angesichts von Zahl und Komplexität der Stufen ist somit auch dieses Verfahren sehr ungünstig.

Aufgabe der vorliegenden Erfindung war es daher, ausgehend von dem Vorprodukt β-Alkoxypropionitril ein Verfahren zu entwickeln, das in wenigen einfachen Schritten direkt zu AMP führt, ohne die Nachteile des Standes der Technik aufzuweisen.

Überraschenderweise zeigte sich nun, daß man 5-Alkoxy-methylpyrimidine der Formel 2, wobei R C₁-C₆-Alkyl bedeutet, durch Umsetzung mit NH₃ in Gegenwart von Katalysatoren unter Substitution des Alkoxyrests mit hoher Selektivität direkt in AMP 1 überführen kann.

C₁-C₆-Alkyl in dem Rest R bedeutet, wenn nicht anders angeben, für sich oder in Kombination z. B. mit Alkoxy, einen geradkettigen, verzweigten, gesättigten oder ungesättigten Rest mit 1 - 6 Kohlenstoffatomen wie z. B. den Methyl-, Ethyl-, Propyl- oder Isopropylrest, bevorzugt den Methylrest.

Gemäß dem erfindungsgemäßen Verfahren wird 2 in einem inerten organischen Lösungsmittel oder in Ammoniak selbst vorgelegt. Bevorzugte Lösungsmittel sind aliphatische oder aromatische organische Solventien, beispielsweise Cycloalkane wie Cyclohexan oder Decalin, oder aber Benzol, Toluol, Xylol, Mesytilen. Die Lösungsmittel können sowohl jeweils allein bzw. im Gemisch untereinander oder mit Ammoniak eingesetzt werden. Vorzugsweise wird 2 in Ammoniak selbst vorgelegt.

Nach Zugabe des Katalysators führt man die Umsetzung in einem Temperaturbereich von etwa 50 - 400°C, bevorzugt bei etwa 180 - 350°C, insbesonders bevorzugt in einem Bereich von 210 - 300°C durch.

Als Katalysatoren werden Lewis- oder Brönstedtsäuren, bevorzugt lewissaure oxidische Verbindungen der Elemente aus den Gruppen IV A oder III B, insbesonders bevorzugt Al₂O₃ eingesetzt.

Ammoniak wird mit 1 - 500 Äquivalenten, bevorzugt 10 - 300, besonders bevorzugt mit 25 - 250 Äquivalenten pro Äquivalent 2 eingesetzt.

Ausgangsmaterial für die Aminierung sind 2-Methyl-4-amino-5-alkoxymethylpyrimidine der Formel 2. Die Synthese von 2 aus den korrespondierenden β-Alkoxypropionitrilen ist im Stand der Technik beschrieben (Pharm. Chem. J. 5 (1971), 8, 495; Khim.-Farm. Zh., 12 (1978), 7, 106).

Dazu wird das β-Alkoxypropionitril zunächst durch Kondensation mit einem C₁-C₆-Alkylformiat in Gegenwart eines Alkalimetallalkoholats oder durch Druckreaktion mit Kohlenmonoxid in einem niederen Alkanol in Gegenwart eines Alkalimetallalkoholats (DE-OS 2107990) in das Alkalimetallenolat des korrespondierenden α-Formyl-β-alkoxy-propionitrils überführt. Daraus wird durch Alkylierung (z.B. mit Dimethylsulfat : R'' = CH₃) der entsprechende Enolether hergestellt. Kondensation mit Acetamidin liefert das 5-Alkoxymethylpyrimidin der Formel 2 (s. Formelschema 3).

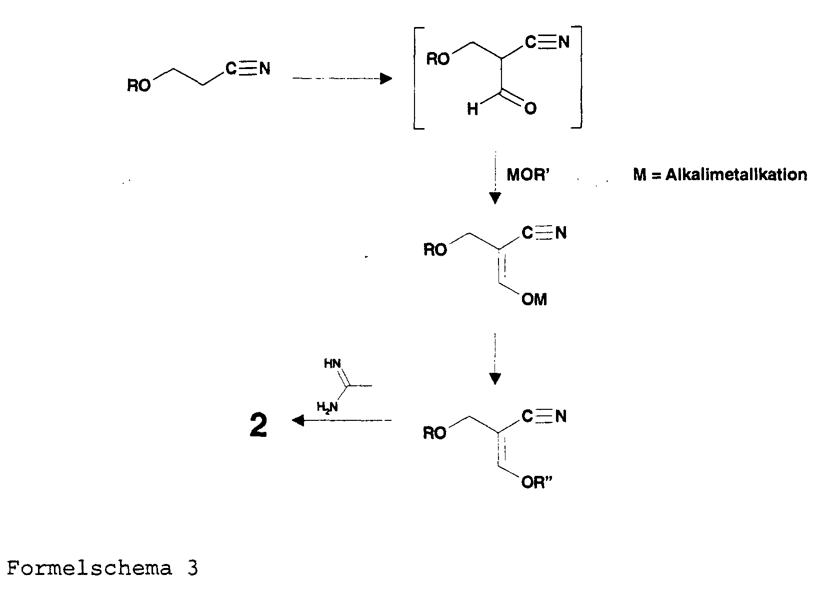

Das erfindungsgemäße Verfahren der direkten Überführung von 2 in AMP (1) ermöglicht somit einen kurzen und attraktiven Zugang zu dieser wertvollen Vitamin B 1-Vorstufe, ausgehend von einem günstigen und gut zu handhabenden Vorprodukt.

Die nachfolgenden Beispiele sollen die Erfindung näher erläutern, ohne sie jedoch darauf einzuschränken.

### Beispiel 1:

In einem 300 ml Autoklaven wurden 1.25 g (8.2 mmol) MMP und 5 g Al₂O₃ (D-10-10, BASF) in 50 ml Toluol vorgelegt. Nach dem Verschließen wurden 30 g (1.76 mol, 215 Äquiv.) Ammoniak zugegeben und für 4 h unter Rühren bei Eigendruck auf 230°C erwärmt. Nach dem Erkalten wurde der Reaktionsaustrag filtriert, in Ethanol aufgenommen und gaschromatographisch analysiert (Tab. 1, Eintrag 1).

### Beispiele 2-9:

In einem 300 ml Autoklaven wurden 1.25 g (8.2 mmol) MMP und 5 g Katalysator in 50 ml Toluol vorgelegt. Nach dem Verschließen wurden 30 g (1.76 mol, 215 Äquiv.) Ammoniak zugegeben und für 4 h unter Rühren bei Eigendruck erwärmt. Nach dem Erkalten wurde der Reaktionsaustrag filtriert, in Ethanol aufgenommen und gaschromatographisch analysiert (Tab. 1).

**Tabelle 1**

| Eintrag | Katalysator | Temp. [°C] | NH₃/MMP [mol/mol] | U_{MMP} [%] | Ausbeute AMP [%] | Selektivität [%] |
|---|---|---|---|---|---|---|
| 1 | Al₂O₃ | 230 | 215 | 87 | 80 | 92 |
| 2 | Al₂O₃ | 230 | 55 | 45 | 39 | 87 |
| 3 | Al₂O₃ | 270 | 215 | 98 | 42 | 43 |
| 4 | Al₂O₃ | 210 | 215 | 40 | 34 | 86 |
| 5 | H₃PO₄ | 270 | 215 | 25 | 23 | 90 |
| 6 | LaPO₄/TiO₂ | 270 | 215 | 55 | 13 | 24 |
| 7 | SiO₂ | 230 | 215 | 2 | 1 | 50 |
| 8 | TiO₂ | 230 | 215 | 41 | 37 | 90 |
| 9 | ZrO₂ | 230 | 215 | 25 | 19 | 78 |

## Patentansprüche

1. Verfahren zur Herstellung von 2-Methyl-4-amino-5-aminomethylpyrimidin der Formel 1 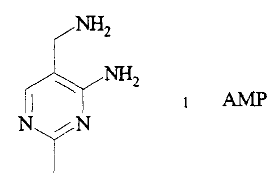 durch Umsetzung von 2-Methyl-4-amino-5-alkoxymethylpyrimidin der Formel 2 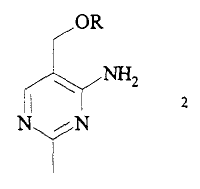
mit R = C₁-C₆-Alkyl
mit Ammoniak in Gegenwart eines Katalysators.

2. Verfahren gemäß Anspruch 1, **dadurch gekennzeichnet, daß** man als Lösungsmittel ein inertes, organisches Solvens einsetzt.

3. Verfahren gemäß einem der Ansprüche 1 oder 2, **dadurch gekennzeichnet, daß** man Ammoniak als Lösungsmittel einsetzt.

4. Verfahren gemäß einem der Ansprüche 1 bis 3, **dadurch gekennzeichnet, daß** man als Katalysatoren Lewis- oder Brönstedtsäuren einsetzt.

5. Verfahren gemäß einem der Ansprüche 1 bis 4, **dadurch gekennzeichnet, daß** man als Katalysatoren lewissaure oxidische Verbindungen der Elemente aus den Gruppen IV A oder III B, bevorzugt Al₂O₃, einsetzt.

6. Verfahren gemäß einem der Ansprüche 1 bis 5, **dadurch gekennzeichnet, daß** man als Katalysator Al₂O₃ einsetzt.

7. Verfahren gemäß einem der Ansprüche 1 bis 6, **dadurch gekennzeichnet, daß** die Verbindung der Formel 2, 2-Methyl-4-amino-5-methoxypyrimidin ist.

8. Verfahren gemäß einem der Ansprüche 1 bis 7, **dadurch gekennzeichnet, daß** nach Zugabe des Katalysators die Umsetzung in einem Temperaturbereich von 50-400°C erfolgt.
